# EUROPEAN PATENT APPLICATION

(11) **EP 4 066 880 A1**
(43) Date of publication of application: **05.10.2022**
(21) Application number: 20894142.7
(22) Date of filing: 18.11.2020
(51) Int. Cl.: A61M 39/22, F16K 11/085, A61M 1/36

(54) **FLOW PATH SWITCHING DEVICE**

(30) Priority: 25.11.2019 JP 2019212580
(71) Applicant: Nipro Corporation, Osaka-shi, Osaka 531-8510 (JP)
(72) Inventor: MASUDA Toshiaki, Osaka-shi, Osaka 531-8510 (JP); KAWABE Minami, Osaka-shi, Osaka 531-8510 (JP)
(74) Representative: Germain Maureau
(86) International application number: PCT/JP2020/043023
(87) International publication number: WO 2021/106716

(57) **Abstract**

A flow path switching device includes an outer cylindrical part (101) having four openings (121) disposed on an outer side surface thereof and equally spaced, a valve (102) rotatably housed in the outer cylindrical part and having two separate flow paths (122) each connecting two adjacent ones of the four openings, and four tubes (103) connected to the four openings, respectively. The tube has a highly stiff portion (136) at an end thereof closer to the outer cylindrical part, the highly stiff portion being stiffer than the other portion of the tube and less stiffer than the outer cylindrical part.

## Description

### TECHNICAL FIELD

The present disclosure relates to flow path switching devices, and more particularly, to a flow path switching device that switches flow paths of an extracorporeal circulation circuit.

### BACKGROUND ART

Various medical procedures are known for removing and returning a fluid such as blood from and to a patient using various tube sets. Among such procedures is, for example, hemodialysis. In hemodialysis, blood is removed from the patient's body through a vascular access, cleansed by a dialyzer, and returned to the patient's body. In hemodialysis, typically, blood is removed from an upstream location of the vascular access, and the cleansed blood is returned to a downstream location of the vascular access. However, in some cases, blood is removed from a downstream location of the vascular access, and the cleansed blood is returned to an upstream location of the vascular access. By thus reversing the direction of the blood flow, various kinds of information such as a vascular access flow rate can be obtained. The information thus obtained can be used to assess a state of the vascular access, and give prior warning about other health problems such as trouble with the vascular access. Accordingly, a procedure such as repair or replacement can be performed on the patient's vascular access that has been used in dialysis for several years.

The direction of blood flow may be reversed by switching around the tubes connected to the vascular access sites. However, switching the tubes around is a complicated procedure and is likely to cause a medical accident. A previously proposed way to switch blood flows without switching the tubes around is to provide a flow path switching device having four ports and a revolving valve having two flow paths connecting the ports, and configured to switch the flow paths by revolving the valve (see, for example, PATENT DOCUMENT 1).

With such a flow rate switching device, the direction of blood flow in extracorporeal circulation can be switched between the normal direction and the reverse direction without switching the tubes around.

### CITATION LIST

### PATENT DOCUMENT

PATENT DOCUMENT 1: International Publication WO2019/070028

### SUMMARY OF THE INVENTION

### TECHNICAL PROBLEM

However, the conventional flow path switching device has the following drawbacks. Firstly, a tube extending toward the patient inevitably intersects with a tube extending toward the dialyzer, disadvantageously leading to a complicated structure. In addition, a port is not aimed at the patient or the dialyzer, and therefore, a tube needs to be guided in the reverse direction, which causes a problem that a kink is likely to occur in a tube attached to the end of a hard port. In addition, unfortunately, it is difficult to intuitively determine in which direction blood is flowing. Furthermore, there is also a problem that blood may stagnate in the flow path switching device. There is also room for improvement in downsizing of the device.

It is an object of the present disclosure to solve at least one of these problems with the conventional flow path switching device.

### SOLUTION TO THE PROBLEM

A flow path switching device according to a first embodiment includes an outer cylindrical part having four openings disposed in an outer side surface thereof and equally spaced, a valve rotatably housed in the outer cylindrical part and having two separate flow paths each connecting two adjacent ones of the four openings, and four tubes connected to the four openings, respectively. The tube has a highly stiff portion at an end thereof closer to the outer cylindrical part, the highly stiff portion being stiffer than the other portion of the tube and less stiff than the outer cylindrical part.

In the flow path switching device of the first embodiment, the tube has a highly stiff portion at an end thereof closer to the outer cylindrical part. Therefore, even when the tube is guided to the patient or the dialyzer, a kink is less likely to occur in the vicinity of a connection portion of the tube and the outer cylindrical part.

In this case, for example, the highly stiff portion may be formed by fitting a sleeve tube on the tube, or by causing the tube to have a great wall thickness.

A flow path switching device according to a second embodiment of the present disclosure includes an outer cylindrical part having four port sections disposed on an outer side surface thereof and equally spaced, a valve rotatably housed in the outer cylindrical part and having two separate flow paths each connecting two adjacent ones of the four port sections, four tubes in communication with the four sections, respectively, and sleeve tubes fixed to the respective port sections, less stiff than the port sections, and extending outward from end portions of the respective port sections.

The flow path switching device of the second embodiment includes the sleeve tubes fixed to the respective port sections, less stiff than the port sections, and extending outward from end portions of the respective port sections. Therefore, even when the tube is guided to the patient or the dialyzer, a kink is less likely to occur in the vicinity of a connection portion of the tube and the port section.

A flow path switching device according to a third embodiment of the present disclosure includes an outer cylindrical part having four openings disposed in an outer side surface thereof and equally spaced, a valve rotatably housed in the outer cylindrical part and having two separate flow paths each connecting two adjacent ones of the four openings, and four tubes connected to the four openings, respectively. A direction in which the tube extends is guided by a guide part. Two of the four tubes that are not adjacent to each other are extended in a first direction along the axis of rotation of the valve, and the other two tubes are extended in a second direction along the axis of rotation of the valve.

When the flow path switching device of the third embodiment is used, two tubes extended to the patient and two tubes extended to the device are extended in the opposite directions along the axis of rotation of the valve, for example. Therefore, the tubes can be extended without intersecting. In addition, the tubes are less likely to be twisted or bent. Furthermore, the length of the tube can be reduced, resulting in a reduction in dead volume.

In the flow path switching device of the third embodiment, the guide part may be a tube guide that is fitted on the tube. With such a configuration, the direction in which the tube extends can be easily set to the first or second direction in use. The guide part may be rotatably fitted on the tube. With such a configuration, the flow path switching device can be packaged with the tubes extended in a direction intersecting with the axis of rotation of the valve so that the bulkiness is reduced. Note that in the case where the guide part is rotatably fitted on the tube, a temporarily stopping part may be provided to limit the relative rotation of the guide part with the tubes directed in the first or second direction. In addition, a fixing section may be provided to fix two tubes extended in the first or second direction with the two tubes disposed adjacent to each other. The fixing section may be removable.

In the flow path switching device of the third embodiment, at least a portion of the guide part may be a port that is directed in the first or second direction. With such a configuration, the tube connected to the port can be directed in a predetermined direction.

A flow path switching device according to a fourth embodiment includes an outer cylindrical part having four openings disposed in an outer side surface thereof and equally spaced, a valve rotatably housed in the outer cylindrical part and having two separate flow paths each connecting two adjacent ones of the four openings, four tubes connected to the four openings, respectively, and a guide part configured to guide at least one of the four tubes such that the at least one tube is extended in a direction along the axis of rotation of the valve. With such a configuration, the direction in which at least one of the four tubes extends in use can be set to a direction along the axis of rotation of the valve, and therefore, the occurrence of a kink in the tube can be prevented or reduced.

A flow path switching device according to a fifth embodiment includes an outer cylindrical part assembly including an outer cylindrical part having four openings disposed in an outer side surface thereof and equally spaced, a valve having a shaft section rotatably housed in the outer cylindrical part and having two separate flow paths each connecting two adjacent ones of the four openings, and a handle section protruding from the shaft section and exposed to the outside of the outer cylindrical part, a first display portion provided on the outer cylindrical part assembly, and a second display portion provided on the handle section. When the flow path switching device transitions from a first state in which one of the four openings is connected to one of two of the four openings adjacent thereto to a second state in which the one of the four openings is connected to the other of the two of the four openings adjacent thereto, the first and second display portions transition from a proximity state in which the first and second display portions are close to each other to a distality state in which the first and second display portions are far away from each other. In the flow path switching device of the fourth embodiment, it is easy to visually intuitively recognize the set state of the flow paths. In addition, if the first and second display portions are a color indication, the states of the first and second display portions can be recognized only by means of identification of color, and therefore, it is easy to confirm the states from a distant location. Note that the first display portion may be provided on the guide part attached to the outer cylindrical part.

A flow path switching device according to a sixth embodiment includes an outer cylindrical part having four openings disposed in an outer side surface thereof and equally spaced, a valve having a shaft section rotatably housed in the outer cylindrical part and having two separate flow paths each connecting two adjacent ones of the four openings, and a handle section protruding from the shaft section and exposed to the outside of the outer cylindrical part, and four tubes connected to the four openings, respectively. The handle section has a direction display portion representing a direction in which a fluid flows. With such a configuration, the state of switching of the flow paths can be intuitively recognized by visually observing the direction in which blood flows.

A flow path switching device according to a seventh embodiment includes an outer cylindrical part having four openings disposed in an outer side surface thereof and equally spaced, a valve rotatably housed in the outer cylindrical part and having two separate flow paths each connecting two adjacent ones of the four openings, and four tubes connected to the four openings, respectively. At least two of the four tubes connected to two of the four opening are partially bonded together with the at least two tubes adjacent to each other. In the flow path switching device of the seventh embodiment, it is easy to handle and extend the tubes due to a reduction in looseness. In addition, unlike the case where a tying member is used, a portion that hides the tube is not produced, and therefore, the risk of overlooking the occurrence of a kink, thrombus, etc., can be reduced.

A flow path switching device according to an eighth embodiment includes an outer cylindrical part having four openings disposed in an outer side surface thereof and equally spaced, and port sections provided for the respective openings, and a valve rotatably housed in the outer cylindrical part and having two separate linear tunnel-shaped flow paths connecting two adjacent ones of the four openings. Each port section protrudes from the outer cylindrical part such that imaginary lines extended from the port sections intersect in the valve as viewed from above. In the flow path switching device of the eighth embodiment, blood is less likely to stagnate.

### ADVANTAGES OF THE INVENTION

In the flow path switching device of the present disclosure, at least one of the problems with conventional flow path switching devices can be solved.

### BRIEF DESCRIPTION OF THE DRAWINGS

[FIG. 1] FIG. 1 is a perspective view illustrating a flow path switching device according to a first embodiment.
[FIG. 2] FIG. 2 is an exploded perspective view illustrating the flow path switching device of the first embodiment.
[FIG. 3] FIG. 3 is a horizontal cross-sectional view illustrating a valve in a first position.
[FIG. 4] FIG. 4 is a horizontal cross-sectional view illustrating a valve in a second position.
[FIG. 5] FIG. 5 is a diagram illustrating an example of connection of a flow path switching device.
[FIG. 6] FIG. 6 is a perspective view illustrating an example of a rotation limiting mechanism of a tube guide.
[FIG. 7] FIG. 7 is a perspective view illustrating a variation of the flow path switching device of the first embodiment.
[FIG. 8] FIG. 8 is an enlarged cross-sectional view illustrating a port section in a flow path switching device according to a second embodiment.
[FIG. 9] FIG. 9 is an enlarged cross-sectional view illustrating a variation of the flow path switching device of the second embodiment.
[FIG. 10] FIG. 10 is a perspective view illustrating an example in which a flow path switching device having a highly stiff portion is used.
[FIG. 11] FIG. 11 is an exploded perspective view illustrating the flow path switching device of the second embodiment.
[FIG. 12] FIG. 12 is a plan view illustrating a valve in a first position in the flow path switching device of the second embodiment.
[FIG. 13] FIG. 13 is a plan view illustrating a valve in a second position in the flow path switching device of the second embodiment.
[FIG. 14] FIG. 14 is a plan view illustrating a flow path switching device according to a third embodiment.
[FIG. 15A] FIG. 15A is a horizontal cross-sectional view illustrating a conventional flow path switching device in a first position.
[FIG. 15B] FIG. 15B is a horizontal cross-sectional view illustrating a conventional flow path switching device in a first position.
[FIG. 16A] FIG. 16A is a diagram illustrating the result of simulation of a conventional flow path switching device in a first position.
[FIG. 16B] FIG. 16B is a diagram illustrating the result of simulation of a conventional flow path switching device in a second position.
[FIG. 17A] FIG. 17A is a horizontal cross-sectional view illustrating a flow path switching device in which a flow path is a linear notch.
[FIG. 17B] FIG. 17B is a vertical cross-sectional view illustrating a flow path switching device in which a flow path is a linear notch.
[FIG. 17C] FIG. 17C is a diagram illustrating the result of simulation of a flow path switching device in which a flow path is a linear notch.
[FIG. 18A] FIG. 18A is a horizontal cross-sectional view illustrating a flow path switching device in which a flow path is an arc notch.
[FIG. 18B] FIG. 18B is a vertical cross-sectional view illustrating a flow path switching device in which a flow path is an arc notch.
[FIG. 18C] FIG. 18C is a diagram illustrating the result of simulation of a flow path switching device in which a flow path is an arc notch.
[FIG. 19A] FIG. 19A is a horizontal cross-sectional view illustrating a flow path switching device in which a flow path is a wavy notch.
[FIG. 19B] FIG. 19B is a vertical cross-sectional view illustrating a flow path switching device in which a flow path is a wavy notch.
[FIG. 19C] FIG. 19C is a diagram illustrating the result of simulation of a flow path switching device in which a flow path is a wavy notch.
[FIG. 20A] FIG. 20A is a horizontal cross-sectional view illustrating a flow path switching device in which a flow path is in the shape of a curved tunnel.
[FIG. 20B] FIG. 20B is a vertical cross-sectional view illustrating a flow path switching device in which a flow path is in the shape of a curved tunnel.
[FIG. 20C] FIG. 20C is a diagram illustrating the result of simulation of a flow path switching device in which a flow path is in the shape of a curved tunnel.
[FIG. 21A] FIG. 21A is a horizontal cross-sectional view illustrating a flow path switching device in which a flow path is in the shape of a linear tunnel.
[FIG. 21B] FIG. 21B is a vertical cross-sectional view illustrating a flow path switching device in which a flow path is in the shape of a linear tunnel.
[FIG. 21C] FIG. 21C is a diagram illustrating the result of simulation of a flow path switching device in which a flow path is in the shape of a linear tunnel.

### DESCRIPTION OF EMBODIMENTS

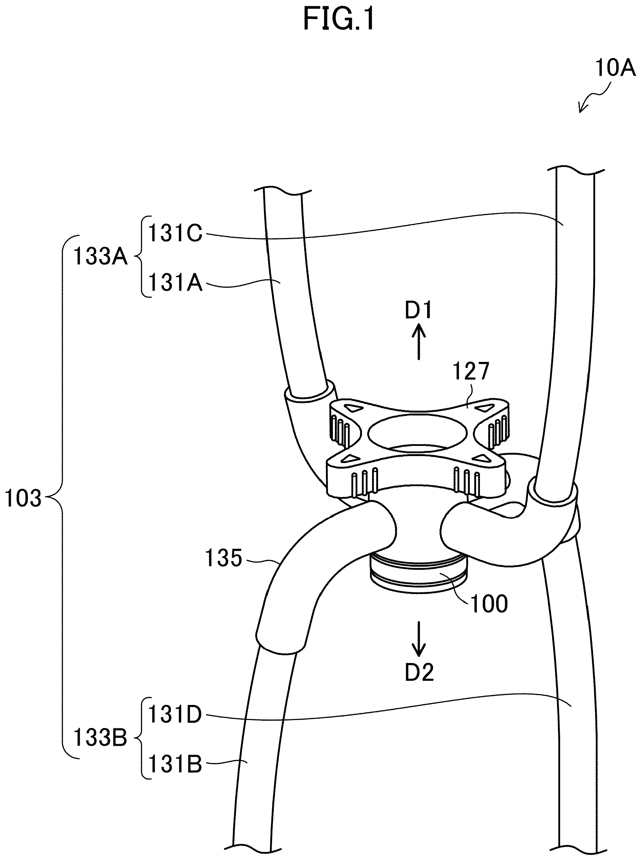

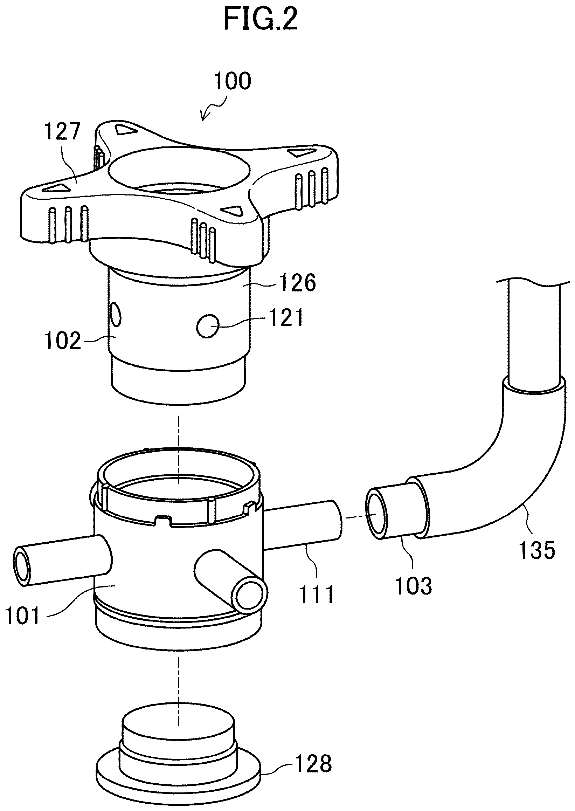

As illustrated in FIGS. 1-4, a flow path switching device 10A according to a first embodiment has a flow path switching device body 100, and four tubes 103 extending from the device body 100. The device body 100 has an outer cylindrical part 101 with four port sections 111 provided on an outer peripheral surface thereof and equally spaced, and a valve 102 that is slidably and rotatably housed in the outer cylindrical part 101.

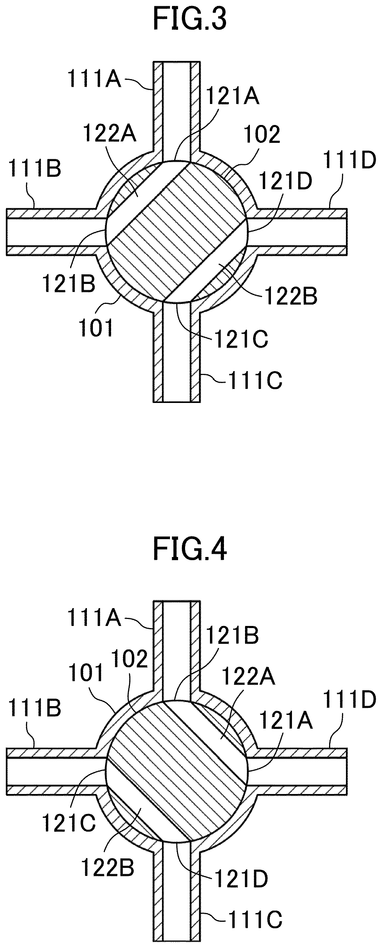

As illustrated in FIGS. 3 and 4, the port sections 111 protrude radially from the outer side surface of the outer cylindrical part 101. Specifically, a first port section 111A, a second port section 111B, a third port section 111C, and a fourth port section 111D are equally spaced with each section apart from an adjacent one by 90°. Therefore, imaginary lines extended from the port sections 111 intersect at a center position of the valve 102 as viewed from above.

The valve 102 has four openings 121 provided in an outer peripheral surface thereof and equally spaced. As illustrated in FIGS. 3 and 4, of the four openings 121, a first opening 121A and a second opening 121B, which are adjacent to each other, are connected together by a first flow path 122A, and a third opening 121C and a fourth opening 121D, which are adjacent to each other, are connected together by a second flow path 122B. The first flow path 122A and the second flow path 122B are each in the shape of a tunnel and are separated from each other, i.e., are independent of each other. The valve 102, which includes the first flow path 122A and the second flow path 122B, has a shaft section 126 that is housed in the outer cylindrical part 101, and a handle section 127 that is exposed to the outside of the outer cylindrical part 101, and can be gripped and rotated. A retainer 128 is attached to the opposite side of the valve 102 from the handle section 127 in order to prevent the valve 102 from coming off the outer cylindrical part 101.

In the flow path switching device 10A of this embodiment, each port section 111 is a hard pipe that protrudes from the outer cylindrical part 101. A tube 103 is fitted on each port section 111. The tube 103 is preferably bonded to the port section in terms of connection strength. The tubes 103 include a first tube 131A connected to the first port section 111A, a second tube 131B connected to the second port section 111B, a third tube 131C connected to the third port section 111C, and a fourth tube 131D connected to the fourth port section 111D.

In the flow path switching device 10A of this embodiment, of the four tubes 103, the first tube 131A and the third tube 131C, which are not adjacent to each other, form a first tube pair 133A, and the second tube 131B and the fourth tube 131D, which are not adjacent to each other, form a second tube pair 133B. The first tube pair 133A (the first tube 131A and the third tube 131C) extends in a first direction D1 (toward the handle section 127) along the axis of rotation of the valve 102, and the second tube pair 133B (the second tube 131B and the fourth tube 131D) extends in a second direction D2 (toward the retainer 128) along the axis of rotation of the valve 102. Note that the tubes included in the first tube pair or the tubes included in the second tube pair may be tied together to be held in a position in a manner that allows the tubes to be untied later. For example, a clip, tape, or ring seat may be provided for holding the tubes included in the first tube pair, or the tubes may be partially bonded together.

In the flow path switching device 10A of this embodiment, when the valve 102 is in a first position as illustrated in FIG. 3, the first opening 121A coincides with the first port section 111A, the second opening 121B coincides with the second port section 111B, the third opening 121C coincides with the third port section 111C, and the fourth opening 121D coincides with the fourth port section 111D. Therefore, the first port section 111A is connected to the second port section 111B by the first flow path 122A, and the third port section 111C is connected to the fourth port section 111D by the second flow path 122B.

As illustrated in FIG. 4, when the valve 102 is rotated by 90° and is thereby changed from the first position into a second position, the first opening 121A coincides with the fourth port section 111D, the second opening 121B coincides with the first port section 111A, the third opening 121C coincides with the second port section 111B, and the fourth opening 121D coincides with the third port section 111C. Therefore, the first port section 111A is connected to the fourth port section 111D by the first flow path 122A, and the second port section 111B is connected to the third port section 111C by the second flow path 122B.

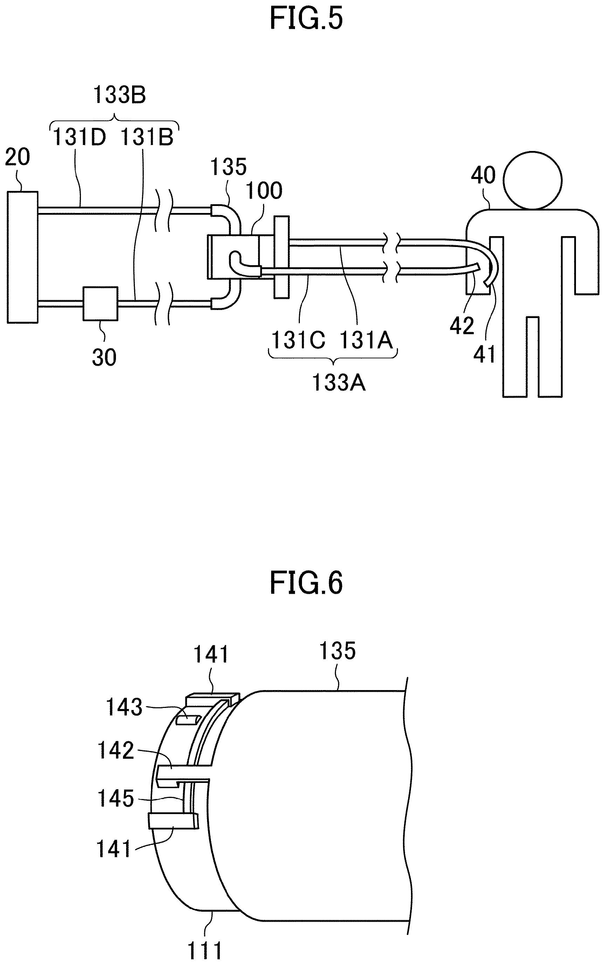

For example, as illustrated in FIG. 5, the first tube 131A, which is included in the first tube pair 133A, is connected to an upstream location 41 of a vascular access provided in a patient 40, and the third tube 131C is connected to a downstream location 42 of the vascular access. The second tube 131B, which is included in the second tube pair 133B, is connected to an arterial port of a dialyzer 20 through a pump 30, and the fourth tube 131D is connected to a venous port of the dialyzer 20. In this case, when the valve 102 is in the first position, the first tube 131A is connected to the second tube 131B, which is connected to the pump 30, and therefore, blood is removed from the upstream location 41 of the vascular access in the patient 40, and blood is returned to the downstream location 42, which is a forward connection. When the valve 102 is in the second position, the third tube 131C is connected to the second tube 131B, which is connected to the pump 30, and therefore, blood is removed from the downstream location 42 of the vascular access in the patient 40, and blood is returned to the upstream location 41, which is a backward connection.

In the flow path switching device 10A of this embodiment, the first tube 131A and the third tube 131C, which are included in the first tube pair 133A and extend toward the patient 40, and the second tube 131B and the fourth tube 131D, which are included in the second tube pair 133B and extend toward the dialyzer 20, are extended in the opposite directions along the axis of rotation of the valve 102. If a tube is extended in a direction intersecting with the axis of rotation, two tubes included in a tube pair extend in the opposite directions, and therefore, at least two of the four tubes need to be turned back in the intended direction. Therefore, a kink is likely to occur, or the user may connect a tube to a wrong site. In PATENT DOCUMENT 1, that problem is overcome by providing a plate having instructions, leading to an increase in size and difficulties in handling. In addition, a tube needs to be turned back in a relatively large arc in order to prevent a kink or the like in the tube, leading to an increase in length of the tube, and an increase in dead volume. Meanwhile, in the flow path switching device 10A of this embodiment, all of the tubes are extended in the intended directions, so that no tubes intersect, and therefore, it is easy to handle and extend the tubes. In addition, for example, a guide part for guiding a tube in an intended direction and a highly stiff part for partially reducing the flexibility of a tube are provided, and therefore, the occurrence of a kink can be prevented in the vicinity of the outer cylindrical part of a tube.

Note that the first tube pair 133A, which extends toward the patient 40, may be extended toward the retainer 128 (second direction), and the second tube pair 133B, which extends toward the dialyzer 20, may be extended toward the retainer 128 (first direction).

In the flow path switching device 10A of this embodiment, a tube guide 135 is fitted on the tube 103, as a guide part for guiding the tube 103 in the first or second direction, at a connection portion of the tube 103 to the port section 111, in order to extend the first tube 131A and the third tube 131C, and the second tube 131B and the fourth tube 131D, in the opposite directions,. In the flow path switching device 10A of this embodiment, the tube guide 135 is a tube or pipe that is harder than the tube 103 and is attached to a portion of the tube 103 or the outer cylinder, and is curved so that the direction is changed by about 90°.

The tube guide 135 is allowed to slide and rotate around the port section 111. Therefore, the direction in which the tube 103 extends can be changed, if necessary, by rotating the tube guide 135. For example, the flow path switching device 10A may be housed in a bag for sterilization with the tube guides 135 set such that the tubes 103 extend in a direction intersecting with the axis of rotation of the valve 102. Therefore, a flat package can be embodied, and the tubes 103 are less likely to be folded or bent in the bag. Meanwhile, when the flow path switching device 10A of this embodiment is used, the tube guides 135 are rotated by about 90°, so that the first tube pair 133A can be extended in the first direction along the axis of rotation of the valve 102, and the second tube pair 133B can be extended in the second direction. Thus, it is easy to handle and extend the tubes 103 in use. Note that the tube guides 135 may not be allowed to slide or rotate around the port section 111.

A limiting mechanism may be provided to limit the range of rotation of the tube guide 135. By providing the limiting mechanism, the tube may be prevented from being mistakenly extended in a wrong direction, or being rotated so much that the tube is twisted. As illustrated in FIG. 6, for example, the limiting mechanism may be implemented by two rotation stopper protrusions 141 provided at a base end portion of the port section 111, and an engagement protrusion 142 provided at a base end portion of the tube guide 135. By preventing the engagement protrusion 142 from passing over the rotation stopper protrusions 141, the range of rotation of the tube guide 135 can be limited between the two rotation stopper protrusions 141. Note that the mechanism for limiting the range of rotation of the tube guide 135 is not limited to the illustrated mechanism. In addition, the mechanism for limiting the range of rotation of the tube guide 135 may be provided if necessary, or may not be provided.

If the tube guide 135 is rotatable, a temporarily stopping part may be provided to limit the rotation of the tube guide 135 with the tube guide 135 extending in the first or second direction.

As illustrated in FIG. 6, for example, a specific example of the temporarily stopping part is a locking protrusion 143 that is provided in front of one of the rotation stopper protrusions 141 and over which the engagement protrusion 142 is allowed to pass. By providing the locking protrusion 143, the feeling of clicking is obtained when the tube guide 135 is rotated to an appropriate position, and therefore, the rotation to the appropriate position can be recognized through sensation. The tube guide 135 can also be easily temporarily stopped at the appropriate position. The locking protrusion 143 may be configured to allow the tube guide 135 to pass over only in a predetermined direction. Note that the mechanism for providing the feeling of clicking and locking at a position is not limited to the illustrated mechanism. In addition, the mechanism for providing the feeling of clicking and locking at a position may be provided if necessary, or may not be provided.

FIG. 6 illustrates an example in which a retainer protrusion 145 is provided to prevent the tube guide 135 from coming off the port section 111. In FIG. 6, the retainer protrusion 145, which is in the shape of a rail connecting the two rotation stopper protrusions 141, prevents a protrusion provided on the engagement protrusion 142 from passing over itself toward the tip end. Note that the retainer mechanism may be a groove in the shape of an annular rail, and is not limited to the illustrated mechanism. In addition, the retainer mechanism may be provided if necessary, or alternatively, for example, the tube guide may be movable along the tube without providing the retainer mechanism.

The tube guide 135 may be cylindrical, and the shape of the tube guide 135 is not limited. For example, the tube guide 135 may have a slit extending in a longitudinal direction thereof in a side surface thereof. It is easy to form the tube guide 135 that has a slit extending in the longitudinal direction. The tube guide 135 may be composed of a single member or multiple members. For example, the tube guide 135 may be a cylindrical member assembled by joining two half members.

The tube guide 135 is preferably harder than the tube 103. Note that the tube guide may be made of vinyl chloride, polyurethane or silicone polyethylene, polypropylene or polyester, or the like.

If the tube guides are allowed to slide and rotate, the tube guides may not be rotated separately, and two of the tube guides 135 may be rotated simultaneously in association with each other. For example, the tube guide 135 attached to the first tube 131A may be linked to the tube guide 135 attached to the third tube 131C, and the tube guide 135 attached to the second tube 131B may be linked to the tube guide 135 attached to the fourth tube 131D, so that the two tube guides linked together can be rotated in the same direction in association with each other, which makes it easy to set the directions in which the tube guides 135 extend.

An example has been described in which the rotatable tube guide 135 is provided so that the tube 103 can extend in a specific direction. The direction in which the tube 103 extends can be set to a specific direction in other ways. For example, a non-elastic flexible hose whose shape can be maintained may be fitted on the tube 103, and may be used as the tube guide 135. Instead of using the tube guide 135, the tube 103 itself may be previously formed in a predetermined shape so that the direction in which the tube 103 extends can be set to a specific direction. In that case, the stiffness of the tube 103 may be higher than normal.

Alternatively, a wire may be buried in the tube 103, or a wire may be fixed to an outer surface of the tube 103, so that the direction in which the tube 103 extends can be set to a specific direction. If a wire is used to set the direction in which the tube 103 extends to a specific direction, the wire may previously have a predetermined shape. Alternatively, if a wire made of a metal or the like that is plastically deformable to some extent is used, the tube 103 can be extended in a predetermined direction by bending the wire in use. Note that the present disclosure is not limited to wires, and a fine board-shaped member or the like may be used.

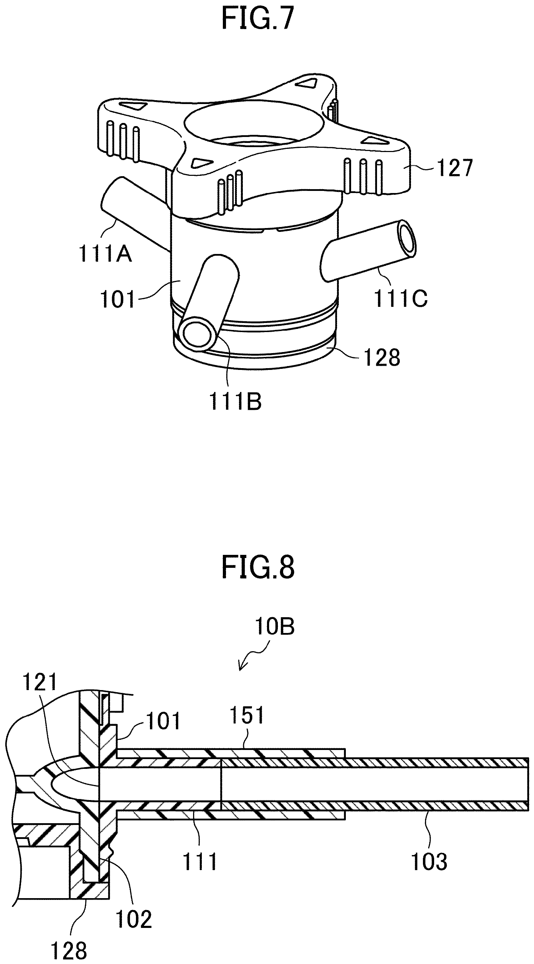

Instead of bending or curving the tube 103 so that the tube 103 extends in a specific direction, the direction in which the port section 111 itself extends may be changed so that the port section 111 serves as a guide section for guiding the tube 103 to extend in a specific direction. For example, as illustrated in FIG. 7, the first port pair of the first port section 111A and the third port section 111C may be inclined and protrude toward the handle section 127 (first direction), while the second port pair of the second port section 111B and the fourth port section 111D may be inclined and protrude toward the retainer 128 (second direction). Note that the modification of the direction of the port section 111 itself may be combined with the use of the tube guide 135. Alternatively, only one of the first port pair and the second port pair may be inclined and protrude. The tube guide 135 may be provided for the port pair that is not inclined so that the directions in which the tubes extend can be set to a specific direction.

FIG. 8 illustrates a portion of a flow path switching device 10B according to the second embodiment. In the flow path switching device 10B, the tube 103 is not fitted on an outer surface of the port section 111, and instead, the tube 103 is connected to the port section 111 by covering the tube 103 and the port section 111 with a sleeve 151 with an end surface of the tube 103 meeting an end surface of the port section 111. An inner surface of the sleeve 151 is firmly bonded to outer surfaces of the tube 103 and the port section 111 by an adhesive. The sleeve 151 protrudes toward the tube, extending over a length of preferably about 5-100 mm, more preferably about 10-80 mm, from an end of the port section 111 in terms of adhesion strength and bulkiness.

The sleeve 151 covering the portion of the tube 103 connecting to the port section 111 can provide a highly stiff portion that is stiffer than the other portion, at an end portion of the tube 103 that is closer to the outer cylindrical part 101 and connects to the port section 111. Specifically, in FIG. 8, the tube 103 and the sleeve 151 form, at the end of the tube 103 closer to the outer cylindrical part, a highly stiff portion that is stiffer than the opposite side (the other portion) from the outer cylindrical part of the tube 103 and is less stiff than the outer cylindrical part. By providing the highly stiff portion, the tube is less likely to be bent at a portion where a kink is likely to occur when the tube is extended in a predetermined direction, so that the occurrence of a kink can be reduced. The occurrence of a kink would deteriorate the flow of blood, leading to stagnation, which in turn increases the risk of production of a thrombus. Thus, the highly stiff portion can reduce the risk of production of a thrombus.

As illustrated in FIG. 8, in the case where the connection portion of the tube 103 to the port section 111 is covered by the sleeve 151, if the tube 103 and the port section 111, which abut each other, have the same inner diameter, there is not a large level difference or step at the connection portion, and therefore, stagnation and hemolysis can be reduced compared to the case where the tube 103 is fitted in or on the port section 111. Furthermore, as illustrated in FIG. 8, in the case where the connection portion at which the tube 103 abuts the port section 111 is covered by the sleeve 151, unlike the case where the tube 103 is fitted on the port section 111, even when the inner pressure of the flow path increases, the tube 103 can be substantially prevented from expanding and coming off the port section 111. In addition, unlike the case where the tube 103 is fitted in the port section 111, blood in the flow path is less likely to come into contact with an adhesive that is used to bond the tube 103 to the port section 111. A silicone-based lubricant or the like for facilitating the rotation of the valve 102 may cause defective bonding of the tube 103 to the port section 111. Unlike the case where the tube 103 is fitted in the port section 111, the influence of the lubricant or the like is reduced. Note that the sleeve 151 of FIG. 8 is preferably elastic. If the sleeve 151 and the tube 103 are flexible, the operability thereof is improved. However, when an external force is accidentally applied to the tube, the tube and the port may no longer have the same cross-sectional shape. In that case, a level difference or step may occur between the tube and the port. If the sleeve 151 is elastic, then when the external force is removed, the cross-sectional shape of the tube quickly recovers to its original shape, so that the tube and the port have the same cross-sectional shape again.

The tube 103 and the port section 111 preferably have the same outer diameters so that there is not a level difference or step therebetween. Because the sleeve 151 can accommodate some level difference or step, the tube 103 and the port section 111 may not have the same outer diameters. A material for the sleeve 151 is preferably one that allows easy bonding of the sleeve 151 to the tube 103 and the port section 111. The material for the sleeve 151 also preferably has an elastic modulus higher than that of the tube 103 in terms of reduction of a kink.

Note that in order to prevent or reduce a kink in the tube 103, a highly stiff portion that is stiffer than the other portion is provided at a portion of the tube 103 that is connected to the port section 111. To this end, the tube 103 may be fitted in or on the port section 111, and the tube 103 and/or the port section 111 may be covered by the sleeve 151. The sleeve 151 may be fitted on both the tube 103 and the port section 111. Alternatively, the sleeve 151 may be fitted on the tube 103 with the sleeve 151 and the port section 111 arranged in series. Alternatively, the sleeve 151 may be fitted on the tube 103 with the sleeve 151 interposed between the port section 111 and the tube 103.

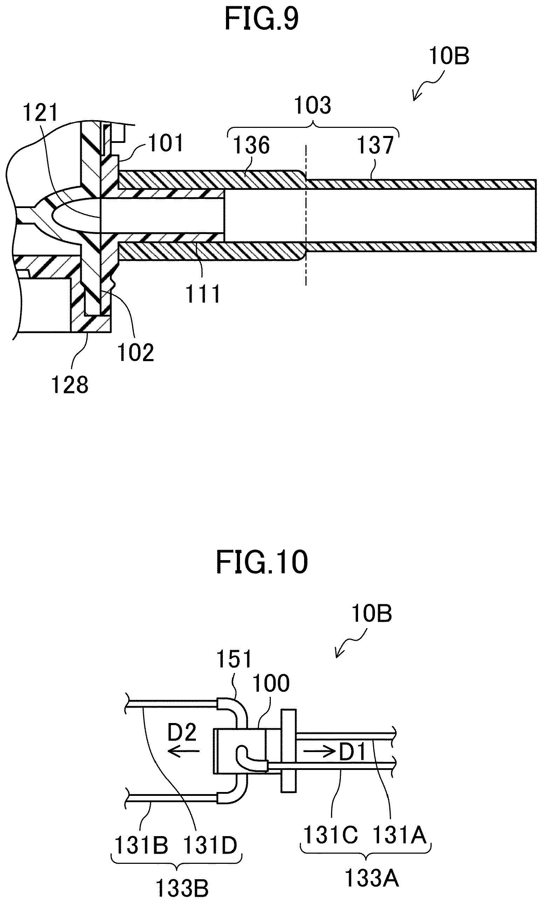

Alternatively, as illustrated in FIG. 9, instead of providing the sleeve, the tube 103 may have a highly stiff portion 136 in the vicinity of the end of the tube 103 closer to the outer cylindrical part 101. In FIG. 9, the tube 103 itself has a greater wall thickness in the vicinity of the connection portion thereof to the port section 111 to increase the stiffness thereof, so that the highly stiff portion 136 is formed. The highly stiff portion 136 is stiffer than that of a normal portion 137 of the tube 103, and may have various configurations. For example, a portion of the tube 103 in the vicinity of the connection portion thereof to the port section 111 may be formed by co-injection molding or burying a wire, whereby the highly stiff portion 136 can be formed. Note that the highly stiff portion is preferably less stiff than the outer cylindrical part 101 in terms of ease of connection to the port section 111 and reduction of occurrence of a kink. In addition, at least a portion of the highly stiff portion is preferably exposed outward of the outer cylindrical part 101.

FIG. 10 illustrates a state that the tubes are extended in predetermined directions in a flow path switching device having a highly stiff portion. In FIG. 10, the highly stiff portion is formed by covering the tube with the sleeve 151. Alternatively, for example, the highly stiff portion may be formed by increasing the wall thickness of the tube and thereby increasing the stiffness of the tube. As illustrated in FIG. 10, when the first tube pair 133A and the second tube pair 133B are extended in opposite directions along the axis of rotation of the valve, the highly stiff portion is curved without collapsing, and therefore, a kink is less likely to occur in each tube. The highly stiff portion may be a typical elastically deformable member, or alternatively, a plastically deformable member that can maintain a shape that is produced when the member is bent.

A first display portion may be provided on an outer cylindrical part assembly including the outer cylindrical part 101, and a second display portion may be provided on the handle section 127. When the flow paths are switched by rotation of the handle section 127, the first and second display portions are switched between a proximity state in which the first and second display portions are close to each other and a distality state in which the first and second display portions are far away from each other. In that case, the connection states of the flow paths can be easily and intuitively recognized by visual observation. If the first and second display portions are represented in different colors, the connection states of the flow paths can be recognized only by identifying the colors, and therefore, can be recognized from a distant location.

For example, a color is given to one of the sleeves 151 attached to the outer cylindrical part 101 so that that sleeve 151 serves as a first display portion, and another color is given to a predetermined portion of the handle section 127 of the valve 102 so that that portion serves as a second display portion. In the first position of FIG. 3, the first and second display portions are close to and overlap each other. In the second position of FIG. 4, the first and second display portions are spaced apart. A plurality of the first display portions and a plurality of the second display portions may be provided instead of a single one. Not only may the first display portion be a colored sleeve, but also the first display portion may be directly provided on the outer cylindrical part, or a certain member may be fixed to the outer cylindrical part.

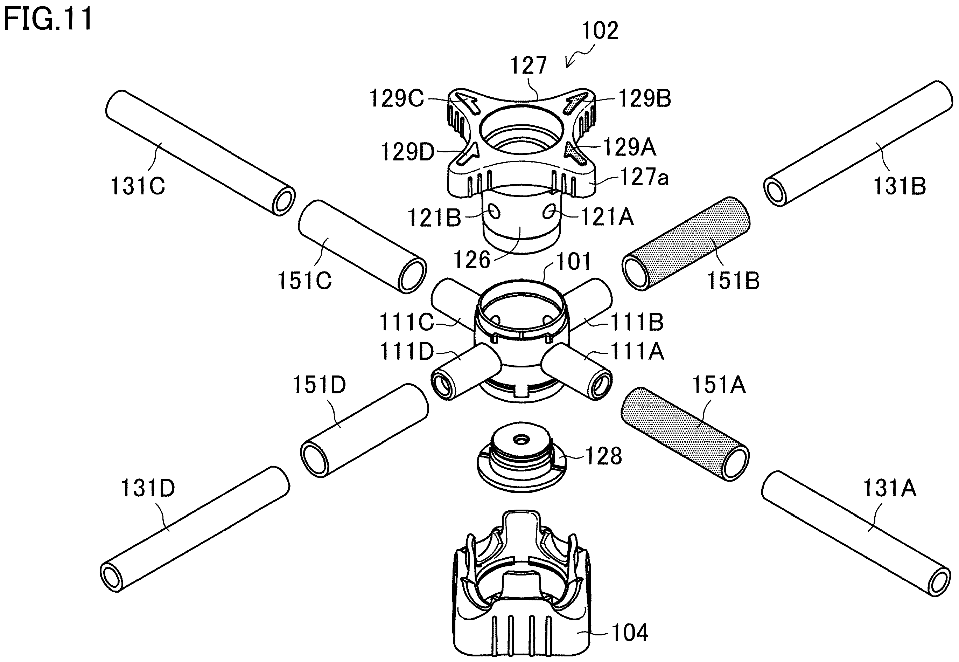

As illustrated in FIGS. 11-13, different colors may be given to the different sleeves 151 attached to the outer cylindrical part 101 so that each sleeve 151 serves as the first display portion. In addition, as illustrated in FIGS. 11-13, the second display portion may be provided on each protrusion of the handle section 127 that is generally in the shape of a cross as viewed from above.

The sleeves 151 given different colors also serve as port section identification indications that facilitate identification of the port sections 111. For example, in the connection illustrated in FIG. 5, the sleeve 151A having a red indication is used for the first tube 131A and the second tube 131B, which are used to remove blood in the forward connection, and the sleeve 151B having a blue indication is used for the third tube 131C and the fourth tube 131D, which are used to return blood in the forward connection. Thus, the red sleeve 151A serves as a first port section identification indication for identifying the first port pair of the first and second port sections, and the blue sleeve 151B serves as a second port section identification indication for identifying the second port pair of the third and fourth port sections. As a result, the functions of each tube and each port section can be easily recognized.

Furthermore, the second display portion provided on the handle section 127 of the valve 102 clearly indicates the positions of the first flow path 122A and the second flow path 122B, and therefore, the connection states of the flow paths can be easily recognized. In the flow path switching device 10B, the handle section 127 is generally in the shape of a cross having four protrusions 127a as viewed from above. The four protrusions 127a of the handle section 127 are provided at positions corresponding to the four openings, respectively, of the valve 102. Therefore, the four protrusions 127a of the handle section 127 serve as an opening position indication indicating the position of an opening.

As the opening position indication, the same color indication as that of the port section identification indication may be used. For example, in FIG. 11, a red indication serving as the first opening position indication is provided on the two protrusions 127a corresponding to the first and second openings at the opposite ends of the first flow path 122A, and a blue indication serving as the second opening position indication is provided on the two protrusions 127a corresponding to the third and fourth openings at the opposite ends of the second flow path 122B. Thus, in the forward direction connection, every pair of a protrusion 127a and a sleeve 151 have the same color indication, while in the backward direction connection, not every pair of a protrusion 127a and a sleeve 151 have the same color indication. Therefore, it can be recognized at a glance whether the connection direction is the forward direction or the backward direction. Note that the first and second opening position indications should be clearly distinguished from each other. The first opening position indication and the first port section identification indication may have different color indications, and the second opening position indication and the second port section identification indication may have different color indications.

Furthermore, if the indication provided on the protrusion 127a also indicates the direction in which blood flows, the connection state can be more intuitively recognized. In FIGS. 11-13, a red inward arrow indication 129A is provided on the protrusion 127a corresponding to the first opening, a red outward arrow indication 129B is provided on the protrusion 127a corresponding to the second opening, a blue outward arrow indication 129C is provided on the protrusion 127a corresponding to the third opening, and a blue inward arrow indication 129D is provided on the protrusion 127a corresponding to the fourth opening.

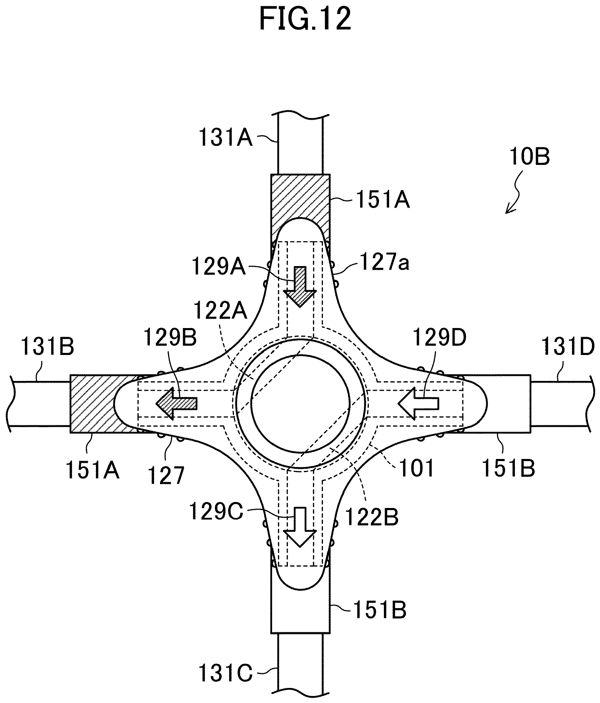

In the circuit connection of FIG. 5, i.e., the first position, which provides the forward connection, as illustrated in FIG. 12 the protrusion 127a having a red indication coincides with the sleeve 151A, which has a red indication, and the protrusion 127a having a blue indication coincides with the sleeve 151B, which has a blue indication. When blood flows from the patient into the first tube 131A, an inward arrow coincides with the position of the first tube 131A, and when blood flows from the dialyzer into the fourth tube 131D, an inward arrow coincides with the position of the fourth tube 131D. When blood flows out toward the dialyzer through the second tube 131B, an outward arrow coincides with the position of the second tube 131B, and when blood flows out toward the patient through the third tube 131C, an outward arrow coincides with the position of the third tube 131C. Therefore, it can be recognized at a glance that removed blood flows in from the first tube 131A, and then is sent out from the second tube 131B, and blood to be returned flows in from the fourth tube 131D, and then flows out into the third tube 131C.

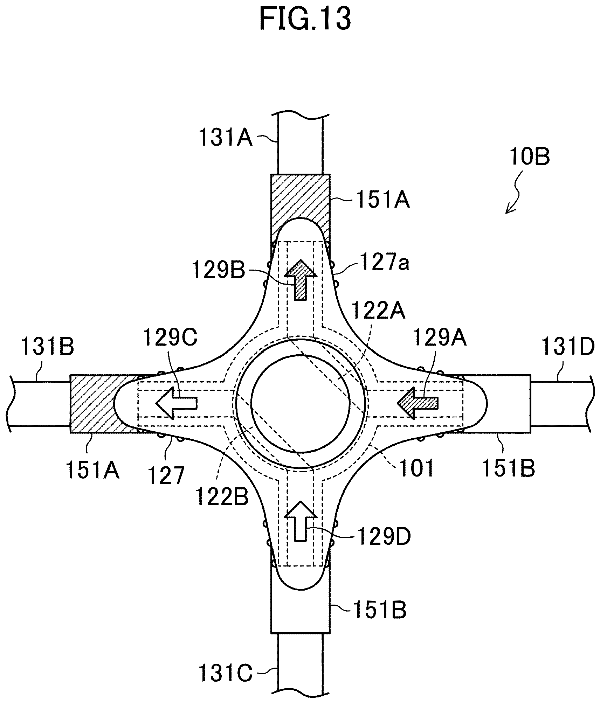

Meanwhile, as illustrated in FIG. 13, when the valve 102 is rotated by 90° into the second position, which provides the backward connection, there is a mismatch between the color given to the sleeve 151 and the color given to the protrusion 127a at the positions of the second tube 131B and the fourth tube 131D. When blood flows from the patient into the third tube 131C, an inward direction coincides with the position of the third tube 131C, and when blood flows from the dialyzer into the fourth tube 131D, an inward arrow coincides with the position of the fourth tube 131D. When blood flows out toward the dialyzer through the second tube 131B, an outward arrow coincides with the position of the second tube 13B, and when blood flows out toward the patient through the first tube 131A, an outward arrow coincides with the position of the first tube 131A. Therefore, it can be recognized at a glance that blood flows in the backward direction, i.e., blood is removed through the normal blood returning line, and blood is returned through the normal blood removing line. Although in this embodiment, there are four arrows for indicating the direction of a flow path, two large arrows may be used to indicate the direction of a flow path. Instead of an arrow, the direction of a flow path may be indicated in other ways.

In the case where the port section identification indication is a color indication provided on the sleeve, the sleeve 151 may be colored entirely or partially. Although in the above example, two colors, i.e., red and blue, are used, other colors may be used. Instead of two colors, four colors may be used. Instead of the color indication, character indications or symbol indications may be used. Color indications, character indications, and symbol indications, etc., may be combined in various ways. Note that the color indication facilitates confirmation from a slightly distant location.

In the above example, all of the protrusions 127a of the handle section 127 have the same length. Alternatively, protrusions having different lengths may be provided to allow easy recognition of the rotational position of the valve 102. If the handle section 127 is in the shape of a flat cross, the handle section 127 is easy to hold, and can be used as an opening position indication indicating the position of the openings. Alternatively, the handle section 127 may be in other shapes. For example, the handle section 127 may be in the shape of a single lever, a flat triangle, or a flat circle. Even in the case of these shapes, an opening position indication can be provided using various techniques.

In this embodiment, the port section identification indications are provided such that one port pair can be distinguished from the other port pair. Note that no indication may be provided to the port section itself. For example, an indication may be provided next to each port section. A first port section identification indication may be provided between the first port section 111A and the second port section 111B, and a second port section identification indication may be provided between the third port section 111C and the fourth port section 111D.

In this embodiment, the opening position indication can indicate where the four openings 121 are located. Note that the opening position indication may not directly indicate the positions of the four openings 121. For example, a first opening position indication may be provided at a middle point of the first flow path 122A, and a second opening position indication may be provided at a middle point of the second flow path 122B. This allows indirect recognition of which of the port sections 111 is connected to which of the four openings 121 through a flow path. In that case, the first and second opening position indications may, for example, be an arrow indication indicating the direction of a fluid flowing in a flow path.

In order to facilitate recognition of the connection state of the port sections, the flow path switching device may be configured so as to allow visual recognition of blood flowing through the flow paths provided in the valve 102. As a technique of allowing visual recognition of the flow path, the thickness of a center portion of the valve 102 closer to the handle section 127 is reduced so that a portion of the flow path can be seen being three-dimensionally bulged, for example. Alternatively, the valve 102 and the handle section 127 may be transparent or translucent. If the valve 102 and the handle section 127 are transparent or translucent so that blood flowing in the flow path can be visually recognized, confirmation of removal of bubbles from the flow path can be facilitated.

Also in the flow path switching device 10B of the second embodiment, the direction in which the first tube pair 133A extends may be set to the first direction along the axis of rotation of the valve 102, and the direction in which the second tube pair 133B extends may be set to the second direction. In that case, instead of or in addition to the sleeve 151, a port section identification indication may be provided on the tube guide 135. The tube guide 135 may also be transparent or translucent so that the port section identification indication provided on the sleeve 151 can be visually recognized. When the tube 103 is extended in a direction along the axis of rotation of the valve 102, the entire handle section 127, which has the identification indications, can be viewed from the direction in which the tube 103 extends, and therefore, it is advantageously easier to confirm switching of the flow paths by the identification indications. Note that the port section identification indications may be provided on the retainer.

In the flow path switching device in which the tube 103 is fitted on or in the port section 111, a port section identification indication and an opening position indication can also be provided.

FIG. 11 illustrates an example in which a lower end portion of the outer cylindrical part 101 is housed in a case 104. The case 104 makes it easy to hold the flow path switching device, resulting in an improvement in operability. Note that the case 104 may be provided if necessary, or may not be provided. Instead of housing the lower end portion of the outer cylindrical part 101 in the case 104, the outer diameter of the lower end portion of the outer cylindrical part 101 may be increased, or a rib is provided on the lower end portion of the outer cylindrical part 101, to make it easy to hold the flow path switching device. In flow path switching devices according to other embodiments and variations, a configuration that makes it easy to hold the flow path switching device, such as a case, may be provided.

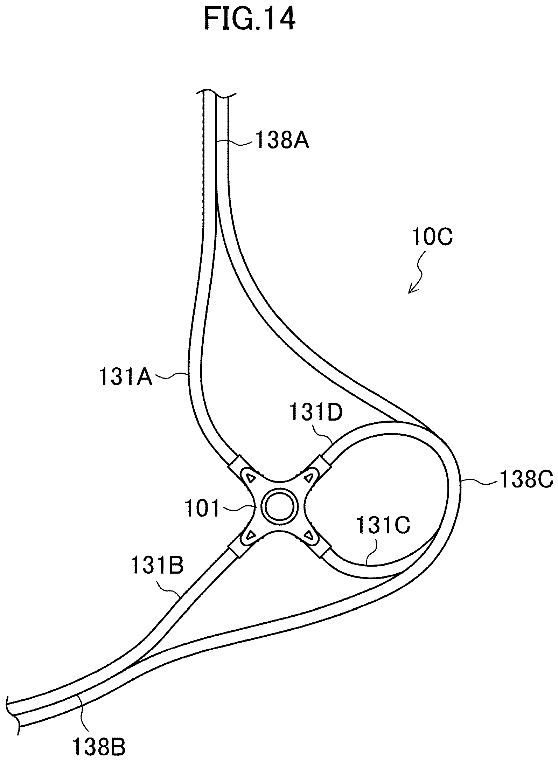

FIG. 14 illustrates a flow path switching device 10C according to a third embodiment. The flow path switching device 10C has a tight bonding section in which tubes 103 are tightly bonded together. FIG. 14 illustrates an example in which there are a first tight bonding section 138A in which the first tube 131A and the third tube 131C, which are a first tube pair, are tightly bonded together, a second tight bonding section 138B in which the second tube 131B and the fourth tube 131D, which are a second tube pair, are tightly bonded together, and a third tight bonding section 138C in which the third tube 131C and the fourth tube 131D, which belong to the different tube pairs, are tightly bonded together.

In the flow path switching device 10C of the third embodiment, which has the tight bonding sections, the tubes 103 support each other, and the positions of the tubes 103 are fixed to some extent. Therefore, a kink is less likely to occur, and the tubes can be fastened together without providing another member.

In the flow path switching device 10C of the third embodiment, two tubes are arranged generally in parallel and firmly bonded together in each tight bonding section. Therefore, the tubes are bonded together across a wide range, and therefore, force is dispersed. As a result, the arrangement and formation of the tubes can be stabilized.

The tubes can be tightly bonded together by adhesion using an adhesive, thermal fusion bonding, ultrasonic fusion, or the like. The tubes may be formed as a double lumen tube. In the case where two tubes are tied together using a tying member or the like, the tying member produces an invisible portion, in which it is likely to overlook the occurrence of a thrombus or the like. In the flow path switching device 10C of the third embodiment, the tubes are tightly bonded together, and therefore, an invisible portion is not produced, so that it is less likely to overlook the occurrence of a thrombus.

Although FIG. 14 illustrates an example in which three tight bonding sections are provided, the arrangement or formation of the tubes may be stabilized by providing only any one or two of the tight bonding sections. For example, the first tight bonding section 138A and the second tight bonding section 138B may be provided.

In the flow path switching device of the second embodiment, in which the tube and the port section are connected together by the sleeve, the tight bonding section may also be provided. In a flow path switching device having a tight bonding section, a port section identification indication and an opening position indication may also be provided. Unless the third tight bonding section 138C is provided, in which two tubes belonging to the different tube pairs are firmly bonded together, the first tube pair of the first tube 131A and the third tube 131C and the second tube pair of the second tube 131B and the fourth tube 131D can be extended in the opposite directions along the axis of rotation of the valve 102.

Note that in the first to third embodiments and the variations, the first flow path 122A and the second flow path 122B of the valve 102 are in the shape of a linear tunnel connecting the two openings 121 together. Alternatively, the first flow path 122A and the second flow path 122B may be in the shape of a curved tunnel connecting the two openings 121. Furthermore, the flow path may not be in the shape of a tunnel, and may be formed by cutting out a portion of a side surface of the valve 102.

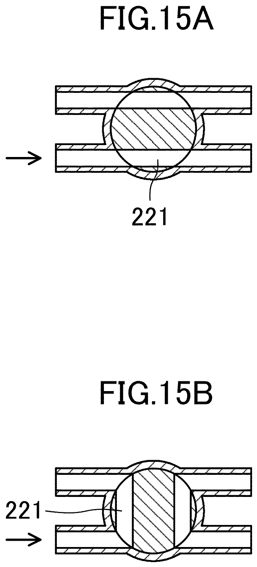

The stagnation in the flow path in the flow path switching device is affected by the position of the port section and the shape of the flow path provided in the valve. As illustrated in FIG. 15A, in a flow path switching device in which each port section is disposed in parallel to one of the port sections adjacent thereto and in series with respect to the other adjacent port section, when the valve is in the first position, the direction in which the port sections connected together by the flow path extends is the same as the direction in which the flow path extends, and therefore, the two port sections and a flow path 221 form a linear flow path. However, as illustrated in FIG. 15B, when the valve is rotated into the second position, the direction in which the port sections extend and the direction in which the flow path extends form an angle of 90°, and therefore, the two port sections, which are disposed in parallel, and the flow path 221 form a flow path having 90°-bent portions.

The stagnation of blood in these two states was assessed by simulation. In the simulation, ANSYS Fluent, which is analysis software, was used to analyze the time it took for a liquid flowing in from the entrance of a flow path to reach positions along the flow path. Note that a k-ε turbulence model was used as the solver, the flow rate at the entrance of the flow path was 200 ml/min, the pressure at the exit of the flow path was 0 Pa, and the fluid model was blood (density: 1060 kg/m³, and viscosity: 0.0033 Pa-s). The outer diameter of the valve was 16.84 mm, and the inner diameter of the port section was 3.3 mm.

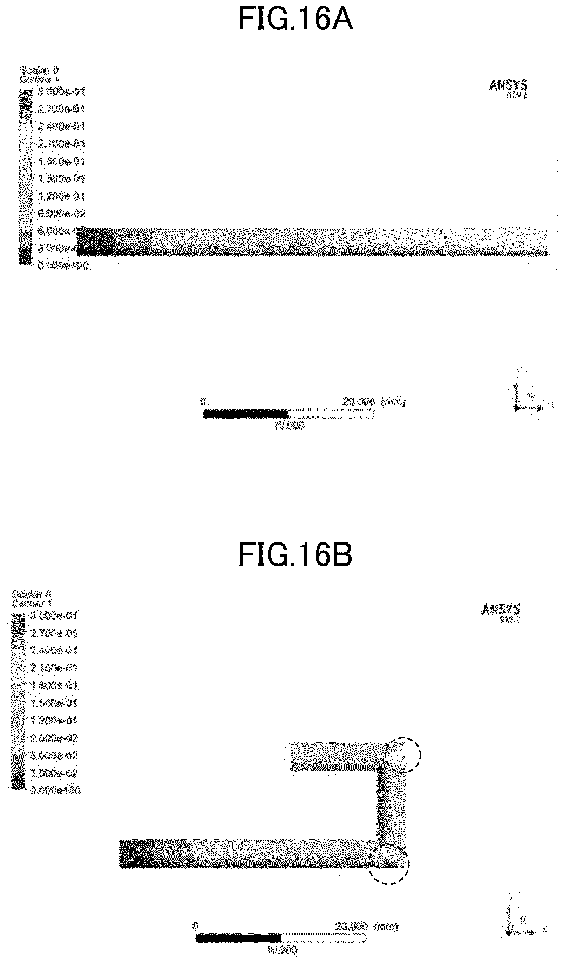

FIGS. 16A and 16B illustrate the results of the analysis. The left side of the drawing sheet indicates the entrance of the flow path, and the right side indicates the exit of the flow path. The time it took for the fluid to reach positions along the flow path is illustrated in a gray scale of 10 levels, i.e., 0-0.03, 0.03-0.06, 0.06-0.09, 0.09-0.12, 0.12-0.15, 0.15-0.18, 0.18-0.21, 0.21-0.24, 0.24-0.27, and 0.27-0.30. Therefore, a portion having a great contrast to the surrounding indicates the occurrence of stagnation.

In the case of the first position of FIG. 15A, as illustrated in FIG. 16A smooth gradations are produced according to the distance from the entrance of the flow path, which indicates that blood almost uniformly flows in the flow path and is less likely to stagnate. Meanwhile, in the case of the second position of FIG. 15B, as illustrated in FIG. 16B a portion having a great contrast is locally produced at an angular portion that is bent, which indicates that the flow is non-uniform and stagnant.

Thus, in the case of a flow path switching device in which two port sections are disposed in parallel, there is a difference in likelihood of stagnation between the directions in which blood flows. Meanwhile, in the case of a flow path switching device in which four port sections are radially disposed and spaced apart from each other by 90°, the shape of the flow path that is taken when the valve is in the first position and the shape of the flow path that is taken when the valve is in the second position are the same, except that the flow paths are rotated, unlike the case where two port sections are disposed in parallel as illustrated in FIGS. 15A and 15B. Therefore, the angle between the direction in which the port section extends and the direction in which the flow path extends in the first position is the same as that in the second position, and therefore, the direction in which blood flows has substantially no influence on the likelihood of stagnation. Therefore, the four port sections are preferably radially disposed on an outer periphery of the outer cylindrical part and spaced apart from each other by 90°.

When the four port sections are radially disposed on an outer periphery of the outer cylindrical part and spaced apart from each other by 90°, and imaginary lines of the port sections protruding from the outer cylindrical part intersect in the valve as viewed from above, an influence of the shape of the flow path formed in the valve on stagnation was assessed by simulation. Note that the conditions for the simulation were the same as when two port sections are disposed in parallel.

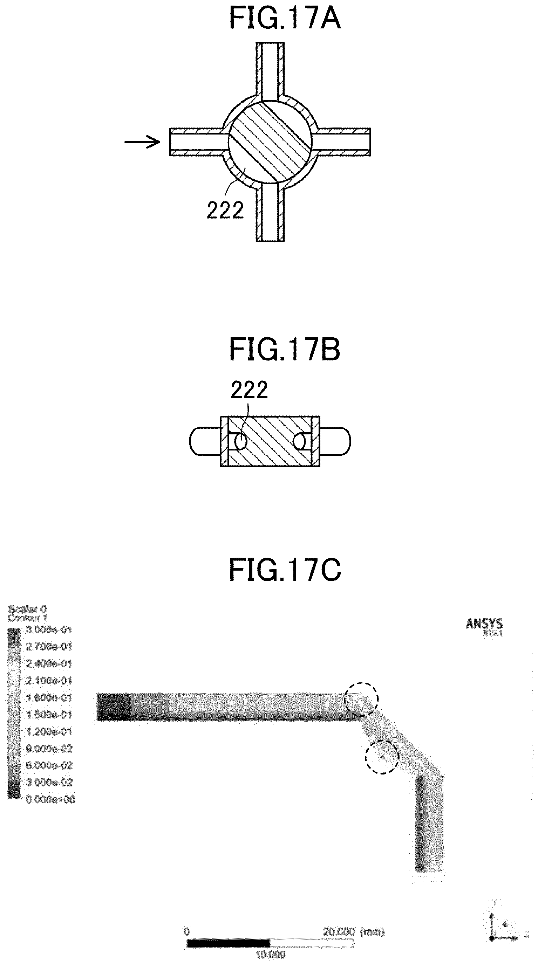

In the case of a flow path 222 formed by linearly cutting a side surface of the valve as illustrated in FIGS. 17A and 17B, as illustrated in FIG. 17C a portion having a great contrast to the surrounding was produced at or near the entrance of the flow path 222 and at a convex portion of a middle of the flow path 222, indicating the occurrence of stagnation. Note that the simulation was conducted, assuming that the flow path 222 thus cut intersects with the radial direction at right angles at a distance of 4.67 mm from the center.

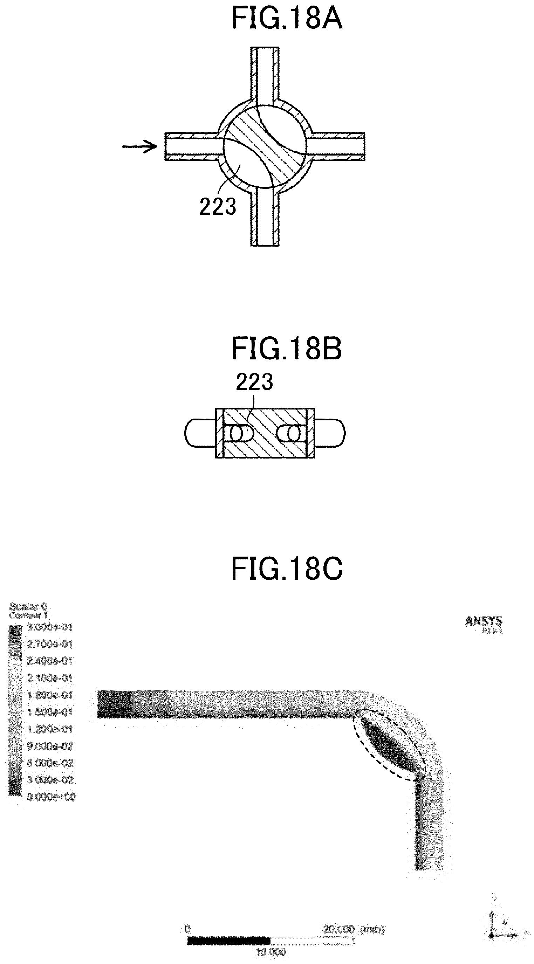

In the case of a flow path 223 formed by cutting a side surface of the valve into the shape of an arc as illustrated in FIGS. 18A and 18B, the flow path has a greater breadth in the vicinity of the middle than when the valve is linearly cut. In that case, as illustrated in FIG. 18C, the broad portion of the flow path 223 has a great contrast compared to the other portion of the flow path 223, which indicates the occurrence of stagnation. Note that in the simulation, the flow path 223 was curved at R11.65.

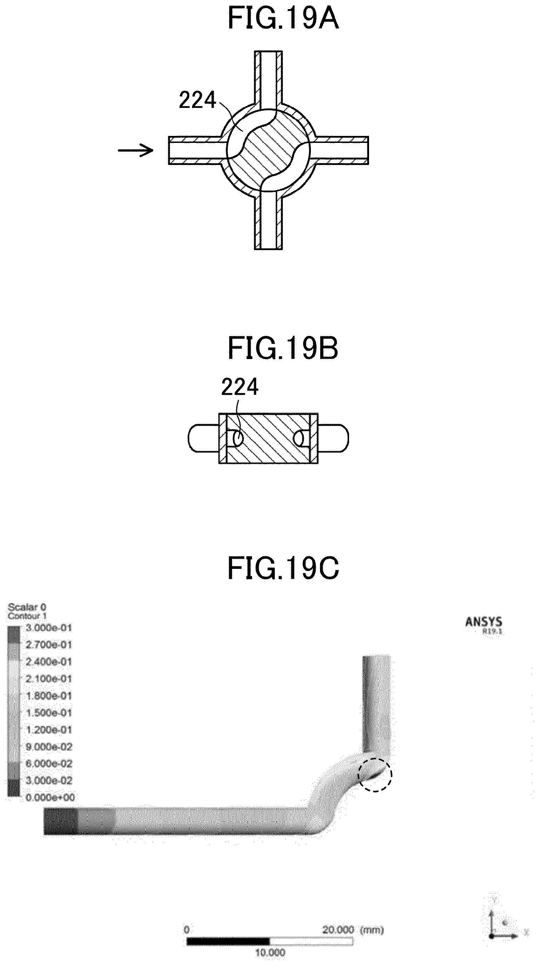

In the case where a flow path 224 is formed by cutting a side surface of the valve into a wavy shape as illustrated in FIGS. 19A and 19B, a portion at or near the exit of the flow path 224 has a great contrast to the surrounding, indicating the occurrence of stagnation as illustrated in FIG. 19C. Note that simulation was conducted, assuming that the flow path 224 has a wavy shape including convex and concave curve segments of R3.3 alternately linked together.

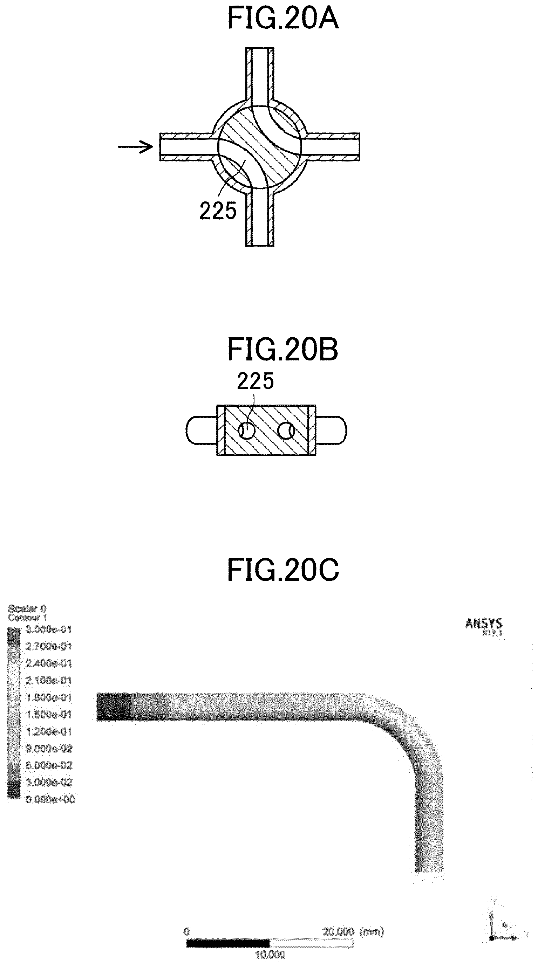

In the case where a flow path 225 is formed in the shape of a curved tunnel connecting two openings in the side surface of the valve as illustrated in FIG. 20A and FIG. 20B, there are some portions having a slight contrast to the surrounding in the flow path 225, and a great stagnation does not occur, as illustrated FIG. 20C. Note that simulation was conducted, assuming that the flow path 225 has an inner diameter of 3.3 mm, and has a center line extending along a curve of R10.

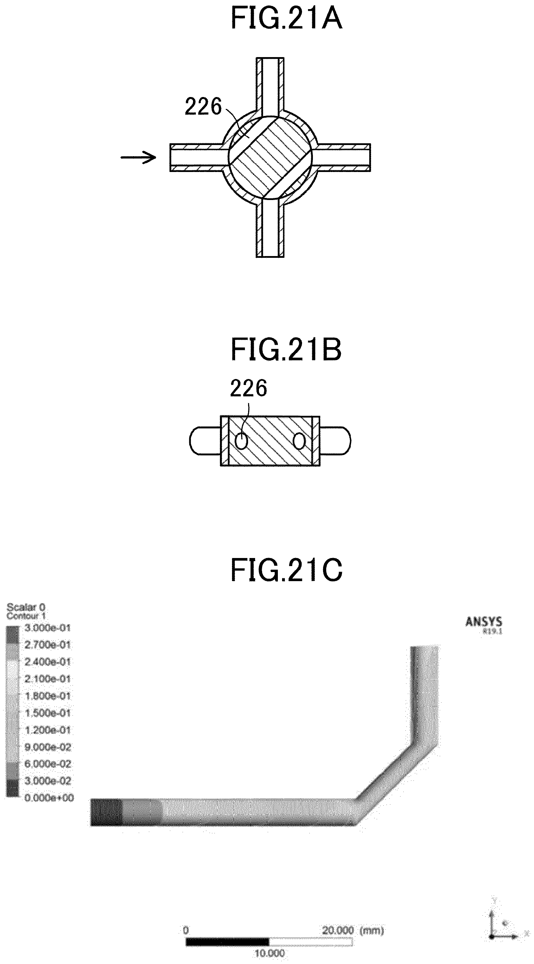

In the case where a flow path 226 is formed in the shape of a linear tunnel connecting two openings in the side surface of the valve as illustrated in FIGS. 21A and 21B, there are some portions having a slight contrast to the surrounding at or near the entrance of the flow path 226, and a great stagnation does not occur as illustrated FIG. 21C. Note that simulation was conducted, assuming that the flow path 226 has an inner diameter of 3.3 mm.

Thus, the flow path in the valve is preferably formed in the shape of a tunnel connecting two openings in terms of reduction of stagnation. In addition, if the tunnel-shaped flow path has a uniform cross-sectional area, blood is likely to flow uniformly, resulting in a further reduction in stagnation. Note that the tunnel-shaped flow path may be either a curved flow path connecting two openings or a linear flow path connecting two openings. Note that in the case of a linear flow path, the flow path and the two port sections connected together by the flow path form an isosceles trapezoid both in the first and second positions, and the angle between the direction in which the port section extends and the direction in which the flow path extends is 135° both in the first and second positions.

In the case of the tunnel-shaped flow path, the side surface of the valve, except for the openings, is interposed between the flow path and the inner surface of the outer cylindrical part. Therefore, the contact area between the inner surface of the outer cylindrical part and the side surface of the valve can be increased, resulting in a larger sealing area. In addition, it is advantageously easy to apply an appropriate pressure to the valve by the outer cylindrical part, so that blood is less likely to leak.

In the case of the tunnel-shape flow path, the flow path and the port section are preferably connected together without a level difference or step. To this end, the openings at the opposite ends of the flow path preferably have the same shape as that of the inner cavity of the port section. For example, in the case of the linear flow path 226, if the openings at the opposite ends of the flow path and the inner cavity of the port section have the same circular shape, the cross-section of the flow path 226 taken along a direction perpendicular to the direction in which the flow path 226 extends is in the shape of an ellipse as illustrated in FIG. 21B.

Although in the flow path switching devices of the above embodiments and variations, the flow path provided in the valve may be in the shape of a tunnel, a tunnel-shaped flow path may be provided in the valve in other types of flow path switching devices so that the influence of stagnation can be reduced. A linear tunnel-shaped flow path connecting two openings is more preferable.

In the flow path switching devices of the above embodiments and variations, materials for the outer cylindrical part 101 and the valve 102 are not particularly limited. For example, polycarbonates, acrylics, polyether sulfones, polyethylene, polypropylene, vinyl chloride, and the like can be used. The outer cylindrical part 101 and the valve 102 may be formed of the same material or different materials. In the case where the valve 102 is compressed, the effect of compressing the valve 102 can be improved if the valve 102 is formed of a material having a hardness lower than that of the outer cylindrical part 101. For example, if the valve 102 is formed of a material having a slightly lower hardness, such as vinyl chloride, the compression effect can be improved, and the valve 102 can be caused to slide and rotate smoothly. Note that the outer cylindrical part 101 and the valve 102 may be formed of materials having the same hardness in terms of reduction of wear of the valve 102.

The inner diameter of the outer cylindrical part 101 is preferably slightly smaller than the outer diameter of the valve 102 so that the valve 102 is compressed. If the outer cylindrical part 101 appropriately compresses the valve 102, fluid leakage is less likely to occur. Although the outer peripheral surface of the valve 102 may be entirely compressed, a portion of the outer peripheral surface in the height direction (axial direction) where the openings are not present may not be compressed. For example, a middle portion where the openings 121 of the valve 102 are present may be compressed, and an upper and a lower end portion of the valve 102 may not be compressed. In that case, fluid leakage is less likely to occur, and the valve 102 can be easily rotated.

In the case where the outer cylindrical part 101 compresses the valve 102, a lubricant is preferably applied to or a lubricating coating is provided on at least one of an inner surface of the outer cylindrical part 101 and an outer surface of the valve 102 so that the valve 102 can be easily rotated. The lubricant or coating may be a silicone-based or fluorine-based one. Note that even in the case where the valve 102 is not compressed, a lubricant may be applied or a lubricating coating may be provided.

The silicone-based lubricant may be one that contains at least one of a first polysiloxane having an amino group at a side chain thereof and a second polysiloxane having an alkoxy group at the opposite ends thereof. The first or second polysiloxane as the lubricant reduces the frictional resistance of the sliding surface, so that the valve 102 can be smoothly rotated. The total contained amount of the first and second polysiloxanes is preferably at least 10 mass% with respect to the total amount of the lubricant, more preferably at least 25 mass%, and may be substantially 100 mass%. Note that the lubricant may consists only of the first polysiloxane or the second polysiloxane.

The first polysiloxane is an organopolysiloxane, which is a type of silicone resin having a siloxane linkage (Si-O-Si) as a main backbone, and has an amino group at a side chain thereof. The first polysiloxane is preferably liquid at room temperature. Because the first polysiloxane has an amino group, which has a high polarity, at a side chain thereof, the first polysiloxane has high affinity to the sliding surface, and therefore, is firmly adsorbed and retained by the sliding surface, resulting in a high lubricating function. The first polysiloxane has at least one of an alkyl group and an aryl group in addition to an amino group. The first polysiloxane may be used alone or in combination with one or more other first polysiloxanes.

The first polysiloxane may have a molecular structure in which no amino group exists at the opposite ends thereof, and an amino group exists only at a side chain(s) thereof. The first polysiloxane may also be a non-crosslinked polysiloxane that has substantially no crosslinked component. In particular, the first polysiloxane is preferably a copolymer of a dialkoxy silane containing an amino group such as 3-aminopropyl dimethoxy methyl silane, and a dialkoxyalkyl silane not having an amino group such as dimethyl dimethoxy silane.

The first polysiloxane may, for example, be a compound represented by formula 1.

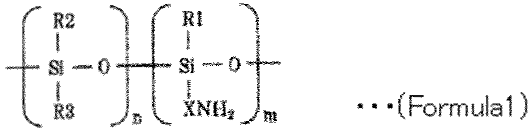

In formula 1, R1-R3 each independently represent an alkyl group having 1-18 carbon atoms or an aryl group. Specific examples of R1-R3 include a methyl group, ethyl group, propyl group, isopropyl group, butyl group, isobutyl group, pentyl group, isopentyl group, hexyl group, cyclopropyl group, cyclohexyl group, phenyl group, naphthyl group, benzyl group, tolyl group, xylyl group, and phenethyl group. R1-R3 are preferably an alkyl group having 1-5 carbon atoms, particularly preferably a methyl group or an ethyl group, and more preferably a methyl group. R1-R3 may be the same or different. For example, R1-R3 may all be a methyl group.

In formula 1, X represents an alkylene group having 1-5 carbon atoms or a group containing at least one heteroatom selected from N or O in the middle of or at a side chain of an alkyl chain. For example, X may be a methylene group, ethylene group, n-propylene group, or n-butylene group, and may preferably be an n-propylene group. X may also be a group having a C-N bond in the middle of an alkyl chain, such as -(CH₂)₃-NH-(CH₂)₂-, or a group having a C-O bond in the middle of an alkyl chain, such as -(CH₂)₃-O-(CH₂)₂-.

In formula 1, the proportion of m (m/(n + m)) is preferably at least 0.03, more preferably at least 0.05, and preferably at most 0.4, more preferably at most 0.3, and even more preferably at most 0.2, in terms of ease of application of the first polysiloxane to the sliding surface and good lubricity of the sliding surface. The kinematic viscosity of the first polysiloxane at 25°C is preferably at least 100 mm²/s, more preferably at least 1,000 mm²/s, and even more preferably at least 5,000 mm²/s. The kinematic viscosity of the first polysiloxane at 25°C is also preferably at most 100,000 mm²/s, more preferably at most 50,000 mm²/s, and even more preferably at most 30,000 mm²/s. A terminal group of the first polysiloxane may be a trialkylsilyl group, particularly preferably a trimethyl silyl group.

The second polysiloxane is an organopolysiloxane having an alkoxy group at the opposite ends thereof, and is preferably liquid at room temperature The second polysiloxane molecules undergo a crosslinking reaction into a higher molecular weight, and are chemically bonded and thereby retained to the sliding surface, whereby the lubricating function is exhibited. The second polysiloxane may be used alone or in combination with one or more other second polysiloxanes.

The second polysiloxane having an alkoxy group at least at the opposite ends thereof undergoes a crosslinking reaction when heated. Therefore, after the second polysiloxane is applied to the sliding surface, the second polysiloxane is heated to undergo a crosslinking reaction, and is thereby chemically fixed to the sliding surface. Alternatively, the heat treatment after application may not be performed, because the second polysiloxane reacts with water in the air at room temperature to be crosslinked.

The second polysiloxane may be a compound represented by formula 2.

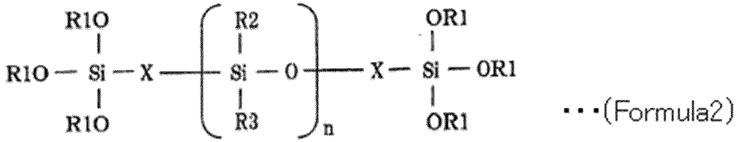

In formula 2, R1 represents an alkyl group having 1-5 carbon atoms, and R2 and R3 each independently represent an alkyl group having 1-18 carbon atoms or an aryl group. R1 is preferably a methyl group, ethyl group, or propyl group, more preferably a methyl group. Specific examples of R2 and R3 include a methyl group, ethyl group, propyl group, isopropyl group, butyl group, isobutyl group, pentyl group, isopentyl group, hexyl group, cyclopropyl group, cyclohexyl group, phenyl group, naphthyl group, benzyl group, tolyl group, xylyl group, and phenethyl group. R2 and R3 are preferably an alkyl group having 1-5 carbon atoms, particularly preferably a methyl group or ethyl group, and more preferably a methyl group. R2 and R3 may be the same or different.

In formula 2, X represents an alkylene group having 1-18 carbon atoms or a group containing at least a C-N bond, C-O bond, or C-Si bond in the middle of or at a side chain of an alkyl chain.

The kinematic viscosity of the second polysiloxane at 25°C is preferably at least 100 mm²/s, more preferably at least 1,000 mm²/s, and even more preferably at least 5,000 mm²/s. The kinematic viscosity of the second polysiloxane at 25°C is also preferably at most 100,000 mm²/s, more preferably at most 50,000 mm²/s, and even more preferably at most 30,000 mm²/s.

The lubricant may contain a polyalkylsiloxane containing an alkyl group having 1-10 carbon atoms in addition to at least one of the first polysiloxane and the second polysiloxane. In particular, the polyalkylsiloxane is preferably polydimethyl siloxane. In the polyalkylsiloxane, a portion of the side chains thereof may be substituted with an aryl group, such as a phenyl group, or hydrogen, or the like, and preferably, does not contain a reactive substituent. The kinematic viscosity of the polyalkylsiloxane at 25°C is preferably at least 50 mm²/s, more preferably at least 80 mm²/s. The kinematic viscosity of the polyalkylsiloxane at 25°C is also preferably at most 100,000 mm²/s, more preferably at most 10,000 mm²/s, and even more preferably at most 1,000 mm²/s.

When the first polysiloxane is used in combination with the polyalkylsiloxane, the volume ratio of the first polysiloxane and the polyalkylsiloxane is preferably 1:10 to 5:1, more preferably 1:9 to 6:4, and even more preferably 2:8 to 4:6. When the second polysiloxane is used in combination with the polyalkylsiloxane, the volume ratio of the second polysiloxane and the polyalkylsiloxane is preferably 1:5 to 10:1, more preferably 5:5 to 9:1, and even more preferably 6:4 to 8:2.

The flow path switching devices of the embodiments and variations may be provided with a click mechanism that allows the user to feel that the valve is brought into the first or second position. The flow path switching devices of the embodiments and variations may also be provided with a rotation limiting mechanism that limits the range of rotation of the valve to the range of 90° between the first and second positions.

The flow path switching devices of the embodiments and variations are not limited to an extracorporeal circulation circuit for dialysis, and may be widely used in extracorporeal circulation circuits and the like that are required to switch flow paths.

### INDUSTRIAL APPLICABILITY

The flow path switching device according to the present disclosure solves at least one of the problems with conventional flow path switching devices, and is useful for extracorporeal circulation circuits and the like that are required to switch flow paths.

### DESCRIPTION OF REFERENCE CHARACTERS

- 20: DIALYZER
- 30: PUMP
- 40: PATIENT
- 41: UPSTREAM LOCATION
- 42: DOWNSTREAM LOCATION
- 100: FLOW PATH SWITCHING DEVICE BODY
- 101: OUTER CYLINDRICAL PART
- 102: VALVE
- 103: TUBES
- 111: PORT SECTIONS
- 111A: FIRST PORT SECTION
- 111B: SECOND PORT SECTION
- 111C: THIRD PORT SECTION
- 111D: FOURTH PORT SECTION
- 121: OPENINGS
- 121A: FIRST OPENING
- 121B: SECOND OPENING
- 121C: THIRD OPENING
- 121D: FOURTH OPENING
- 122A: FIRST FLOW PATH
- 122B: SECOND FLOW PATH
- 126: SHAFT SECTION
- 127: HANDLE SECTION
- 127a: PROTRUSIONS
- 128: RETAINER
- 129A: ARROW INDICATION
- 129B: ARROW INDICATION
- 129C: ARROW INDICATION
- 129D: ARROW INDICATION
- 131A: FIRST TUBE
- 131B: SECOND TUBE
- 131C: THIRD TUBE
- 131D: FOURTH TUBE
- 133A: FIRST TUBE PAIR
- 133B: SECOND TUBE PAIR
- 135: TUBE GUIDE
- 136: HIGHLY STIFF PORTION
- 137: NORMAL PORTION
- 138A: FIRST TIGHT BONDING SECTION
- 138B: SECOND TIGHT BONDING SECTION
- 138C: THIRD TIGHT BONDING SECTION
- 141: ROTATION STOPPER PROTRUSION
- 142: ENGAGEMENT PROTRUSION
- 143: LOCKING PROTRUSION
- 145: RETAINER PROTRUSION
- 151: SLEEVE
- 151A: SLEEVE
- 151B: SLEEVE
- 221: FLOW PATH
- 222: FLOW PATH
- 223: FLOW PATH
- 224: FLOW PATH
- 225: FLOW PATH
- 226: FLOW PATH

## Claims

1. A flow path switching device comprising:
an outer cylindrical part having four openings disposed in an outer side surface thereof and equally spaced;
a valve rotatably housed in the outer cylindrical part and having two separate flow paths each connecting two adjacent ones of the four openings; and
four tubes connected to the four openings, respectively,
wherein
the tube has a highly stiff portion at an end thereof closer to the outer cylindrical part, the highly stiff portion being stiffer than the other portion of the tube and less stiffer than the outer cylindrical part.

2. The flow path switching device of claim 1,
wherein
the highly stiff portion is a portion of the tube on which a sleeve tube is fitted.

3. A flow path switching device comprising:
an outer cylindrical part having four port sections disposed on an outer side surface thereof and equally spaced;
a valve rotatably housed in the outer cylindrical part and having two separate flow paths each connecting two adjacent ones of the four port sections;
four tubes in communication with the four sections, respectively; and
sleeve tubes fixed to the respective port sections, less stiff than the port sections, and extending outward from end portions of the respective port sections.

4. A flow path switching device comprising:
an outer cylindrical part having four openings disposed in an outer side surface thereof and equally spaced;
a valve rotatably housed in the outer cylindrical part and having two separate flow paths each connecting two adjacent ones of the four openings; and
four tubes connected to the four openings, respectively,
wherein
a direction in which the tube extends is guided by a guide part, and
two of the four tubes that are not adjacent to each other are extended in a first direction along the axis of rotation of the valve, and the other two tubes are extended in a second direction along the axis of rotation of the valve.

5. The flow path switching device of claim 4, wherein
the guide part is a tube guide that is fitted on the tube.

6. The flow path switching device of claim 4, wherein
the guide part has a port that is provided at the opening and is connected to the tube, and
at least a portion of the port is directed in the first or second direction.

7. A flow path switching device comprising:
an outer cylindrical part having four openings disposed in an outer side surface thereof and equally spaced;
a valve rotatably housed in the outer cylindrical part and having two separate flow paths each connecting two adjacent ones of the four openings;
four tubes connected to the four openings, respectively; and
a guide part configured to guide at least one of the four tubes such that the at least one tube is extended in a direction along the axis of rotation of the valve.

8. A flow path switching device comprising:
an outer cylindrical part assembly including an outer cylindrical part having four openings disposed in an outer side surface thereof and equally spaced;
a valve having a shaft section rotatably housed in the outer cylindrical part and having two separate flow paths each connecting two adjacent ones of the four openings, and a handle section protruding from the shaft section and exposed to the outside of the outer cylindrical part;
a first display portion provided on the outer cylindrical part assembly; and
a second display portion provided on the handle section, wherein
when the flow path switching device transitions from a first state in which one of the four openings is connected to one of two of the four openings adjacent thereto to a second state in which the one of the four openings is connected to the other of the two of the four openings adjacent thereto, the first and second display portions transition from a proximity state in which the first and second display portions are close to each other to a distality state in which the first and second display portions are far away from each other.

9. A flow path switching device comprising:
an outer cylindrical part having four openings disposed in an outer side surface thereof and equally spaced;
a valve having a shaft section rotatably housed in the outer cylindrical part and having two separate flow paths each connecting two adjacent ones of the four openings, and a handle section protruding from the shaft section and exposed to the outside of the outer cylindrical part; and
four tubes connected to the four openings, respectively, wherein
the handle section has a direction display portion representing a direction in which a fluid flows.

10. A flow path switching device comprising:
an outer cylindrical part having four openings disposed in an outer side surface thereof and equally spaced;
a valve rotatably housed in the outer cylindrical part and having two separate flow paths each connecting two adjacent ones of the four openings; and
four tubes connected to the four openings, respectively; wherein
at least two of the four tubes connected to two of the four opening are partially bonded together with the at least two tubes adjacent to each other.
